# EUROPÄISCHE PATENTSCHRIFT

(11) **EP 3 148 461 B1**
(45) Veröffentlichungstag und Bekanntmachung des Hinweises auf die Patenterteilung: **29.08.2018**
(21) Anmeldenummer: 15725571.2
(22) Anmeldetag: 21.05.2015
(51) Int. Cl.: A61B 17/80

(54) **FIXIERUNGSVORRICHTUNG FÜR KNOCHENFRAKTUREN**
FIXATION DEVICE FOR BONE FRACTURES
DISPOSITIF DE FIXATION POUR DES FRACTURES OSSEUSES

(30) Priorität: 27.05.2014 DE 102014107497
(43) Veröffentlichungstag der Anmeldung: 05.04.2017
(73) Patentinhaber: Hipp Medical AG, 78600 Kolbingen (DE)
(72) Erfinder: HIPP, Markus, 78600 Kolbingen (DE); WIRTH, André, 78600 Kolbingen (DE)
(74) Vertreter: Kuhnen & Wacker Patent- und Rechtsanwaltsbüro PartG mbB
(86) Internationale Anmeldenummer: PCT/EP2015/061319
(87) Internationale Veröffentlichungsnummer: WO 2015/181057

(56) Entgegenhaltungen:
- WO-A2-2004/045389
- WO-A2-2008/048684
- DE-B3-102012 105 123
- US-A1- 2009 082 813

## Beschreibung

Die vorliegende Erfindung betrifft eine Fixierungsvorrichtung für Knochenfrakturen nach Anspruch 1.

Es ist bekannt, dass bei einer unzureichenden Ruhigstellung einer Knochenfraktur eine Kallusbildung, d.h. eine schwielenartige Verdickung der Bruchenden aus überwachsendem Knochengewebe, auftreten kann. Um eine solche indirekte Frakturheilung über einen Kallus zu vermeiden, werden Knochenplatten verwendet, die auf der Außenfläche eines gebrochenen Knochens aufgebracht und befestigt werden, damit die Bruchstelle während des Heilungsprozesses fixiert ist.

Für derartige Anwendungen zur Versorgung von Knochenfrakturen sind aus dem Stand der Technik zumeist starre, flächig ausgebildete, metallische Knochenplatten mit Bohrungen bekannt, die an den gegenüberliegenden Seiten einer Fraktur verschraubt werden. Zur Befestigung solcher Fixierungen werden üblicherweise sogenannte Corticalisschrauben verwendet. Diese werden in das äußere Knochengewebe, die sogenannte Corticalisschicht, welche die höchste Festigkeit des Knochens aufweist, eingeschraubt. Die Fixierung stellt sicher, dass sich die Bruchenden nicht gegeneinander bewegen können und sich neugebildetes Knochengewebe belastungsfrei anlagern kann.

Zudem wird angenommen, dass der Heilungsprozess einer Fraktur günstig beeinflusst werden kann, wenn eine Kompression auf die zusammengefügte Bruchstelle aufgebracht wird. Hierdurch wird eine besonders enge Adaption, d.h. ein geringes Spaltmaß, über welches die Bruchenden wieder aufeinander zu wachsen, bewirkt.

Aus dem Stand der Technik ist eine Vielzahl verschiedener Ansätze zum Ruhigstellen einer Knochenfraktur entnehmbar.

Dabei hat auch die Anmelderin, die sich intensiv mit einer Vielzahl medizinischer Gerätschaften befasst, Entwicklungen im Bereich der Knochenplatten bzw. Fixierungsvorrichtung für Knochenfrakturen getätigt.

So offenbart beispielsweise die DE 10 2011 001 016 A1 der Anmelderin ein Knochenplatten-Modularsystem, bei welchem eine Mehrzahl von Spannelementen, die aus einem hohlen Konturkörper bestehen, nach Art einer Nut-und-Feder Verbindung derart miteinander verbunden wird, dass sie eine durch den Konturkörper hindurchsteckbare Schraube oder dergleichen umschließen. Durch die Kombination mehrerer Spannelemente können diese zu einer Kette oder sternförmig oder in einer beliebigen Kombination aus Ketten- und Sternanordnungen angeordnet werden. Jedoch ist es nicht möglich, die relative Ausrichtung der einzelnen Spannelemente zueinander zu fixieren, wodurch es vereinzelt zu einer Verschiebung der Spannelemente kommen kann, was unerwünscht ist.

Um dieses Problem zu beheben hat die Anmelderin eine andere Ausführungsform einer Fixierungsvorrichtung für Knochenfrakturen vorgeschlagen. Diese aus der DE 10 2012 105 123 A1 bekannte Fixierungsvorrichtung umfasst ebenfalls eine Mehrzahl von aus einem hohlen Konturkörper bestehenden Spannelementen, an deren in Längsrichtung des Spannelements voneinander beabstandeten Enden Aufnahmen ausgebildet sind, in welche Befestigungsmittel eingefügt werden können. Die in der DE 10 2012 105 123 A1 offenbarte Fixierungsvorrichtung schlägt vor, um die einzelnen Spannelemente, abhängig von der Art und Komplexität der zu behandelnden Fraktur, in einer entsprechenden Winkellage zueinander zu fixieren, eine Innenverzahnung in den Aufnahmen auszubilden und, vor der Befestigung der Spannelemente am Knochen vermittels entsprechender Schrauben, die miteinander verbundenen und entsprechend ausgerichteten Spannelemente durch das Einbringen einer mit einer Außenverzahnung versehene Hülsen passgenau in die Aufnahmen zu verbinden. Durch die Kombination der Hülsen und Spannelemente miteinander wird die Winkellage fixiert und die derart zusammengesetzte Fixierungsvorrichtung kann an den Bruchenden mittels entsprechender Schrauben befestigt werden.

Eine weitere Ausführungsform von Knochenplatten bzw. Fixierungsvorrichtungen für Knochenfrakturen aus dem Hause der Anmelderin ist bekannt aus der DE 10 2011 001 018 A1, die ein Spannelement für Fixierungsvorrichtungen offenbart, das im Wesentlichen aus einer flexiblen und im Wesentlichen nicht plastisch verformbaren Endlosschleife besteht, durch die wenigstens zwei Cortikalisschrauben hindurchsteckbar sind, und das ein Streckelement mit einer veränderbaren Länge aufweist, das in die Endlosschleife derart einsetzbar und innerhalb der Endlosschleife derart einspreizbar ist, dass zum Aufbringen von aufeinander zu gerichteten Kräften auf die Befestigungsmittel vermittels des Streckelements eine vorbestimmte Zugspannung über den Umfang der Endlosschleife erzeugbar ist.

Die DE 10 2012 105 125 A1 aus dem Hause der Anmelderin offenbart schließlich eine Knochenplatte mit Gelenken, die eine relative Positionsänderung der einzelnen Abschnitte der Knochenplatte zueinander erlauben.

Derlei aus dem Stand der Technik bekannte Knochenplatten bzw. Fixierungsvorrichtungen weisen dabei insbesondere aufgrund der vielseitigen Anforderungen dieser besonders sensiblen Anwendung einen komplexen Aufbau aus einer Vielzahl verschiedener Bauteile auf, so dass die Herstellung derartiger "Systeme" entsprechend komplex und kostenintensiv ist.

Somit wird bislang in der medizinischen Versorgung lediglich ein begrenzter Kreis an Patienten bzw. an Frakturarten durch die vorteilhafte Anwendung einer kompressionserzeugenden Fixierung begünstigt.

Der Erfindung liegt daher die Aufgabe zugrunde, eine Fixierungsvorrichtung für Knochenfrakturen bereitzustellen, die besonders kostengünstig, insbesondere in Großserienfertigung, herstellbar ist, und geeignet ist, den Anwendungsbereich kompressionserzeugender Fixierungen von Knochenbrüchen zu erweitern.

Die Aufgabe wird durch die Fixierungsvorrichtung für Knochenfrakturen gemäß Anspruch 1 gelöst. Vorteilhafte Ausführungsformen sind Gegenstand der Unteransprüche.

Erfindungsgemäß wird hierbei eine Fixierungsvorrichtung für Knochenfrakturen vorgeschlagen, aufweisend: wenigstens zwei Spannelemente, wenigstens zwei Befestigungsmittel, die, vorzugsweise im Bereich von Bruchenden, in den Knochen einbringbar sind, sowie wenigstens zwei Hülsenelemente, wobei ein jedes Spannelement aufweist: einen in Draufsicht hohlen, eine umlaufende Wandung aufweisenden, Konturkörper mit zwei einander in Breitenrichtung des Spannelements gegenüberliegenden Seitenflanken, die, in Draufsicht betrachtet, von einem gedachten Mittelpunkt des Spannelements bogenförmig nach außen gerichtet sind, wobei die Seitenflanken nahtlos in Y-förmige Verbindungsknoten übergehen, die in einstückig angeformte, in Draufsicht betrachtet zu dem gedachten Mittelpunkt des Spannelements bogenförmig nach innen gerichtete, Aufnahmeabschnitte auslaufen, wenigstens zwei voneinander beabstandete, sich vorzugsweise in Längsrichtung des Spannelements gegenüberliegende, ringförmige Aufnahmen, die zur Aufnahme der Hülsenelemente und/oder der Befestigungsmittel dienen, die jeweils durch die Aufnahmen hindurchsteckbar sind, wobei die Aufnahmeabschnitte integraler Bestandteil der ringförmige Aufnahmen sind, so dass diese einstückig mit dem Konturkörper ausgestaltet sind und ein Hülsenelement und/oder Befestigungsmittel umschließen, und wobei durch eine Verknüpfung mehrerer Spannelemente, Hülsenelemente und/oder Befestigungsmittel diese zu einer Kette angeordnet werden können; wobei die umlaufende Wandung des Konturkörpers eines jeden Spannelements wenigstens abschnittsweise im Bereich der Seitenflanken federelastisch verformbar ist, wobei ein jedes Spannelement punktsymmetrisch in Breitenrichtung bzw. spiegelsymmetrisch zur Längsrichtung des Spannelements ausgebildet ist und die voneinander, vorzugsweise in Längsrichtung des Spannelements gegenüberliegenden, beabstandeten Aufnahmen derart ausgestaltet sind, dass sich eine Aufnahme, vorzugsweise ausgehend von einer gedachten Mittellinie eines jeden Spannelements in Längsrichtung, in Richtung zur Außenkante des Konturkörpers des Spannelements in Höhenrichtung des Konturkörpers nach oben erstreckt, und sich die andere Aufnahme, vorzugsweise ausgehend von der gedachten Mittellinie eines jeden Spannelements in Längsrichtung, in Richtung zur Außenkante des Konturkörpers des Spannelements in Höhenrichtung des Konturkörpers nach unten erstreckt, wobei eine jede ringförmige Aufnahme an ihrer zur gedachten Mittellinie eines jeden Spannelements in Längsrichtung weisende Kontaktfläche eine Verzahnung aufweist, wobei einzelne Spannelemente derart miteinander verbunden werden können, dass die sich, vorzugsweise ausgehend von der gedachten Mittellinie eines jeden Spannelements in Längsrichtung, in Richtung zur Außenkante des Konturkörpers des Spannelements in Höhenrichtung des Konturkörpers nach oben oder unten erstreckenden Aufnahmen derart übereinander zu liegen kommen, dass die zur gedachten Mittellinie des Spannelements weisenden Kontaktflächen der Aufnahmen einander, im wesentlichen deckungsgleich, zugewandt sind, und wobei, durch das miteinander in Eingriff bringen der an den jeweils zur gedachten Mittellinie eines jeden Spannelements in Längsrichtung weisenden, einander zugewandten Kontaktflächen der Aufnahmen ausgebildeten Verzahnung, die Spannelemente in einer vorbestimmten Winkellage zueinander ausrichtbar sind.

Vermittels der erfindungsgemäßen Fixierungsvorrichtung ist es vorteilhaft möglich, selbst komplizierte Brüche, beispielsweise im Schädel- oder Kieferbereich auszurichten, um den Heilungsverlauf positiv zu beeinflussen. Hierbei kann, durch die federelastische Verformbarkeit der einzelnen Spannelemente, ein durch das Material der Spannelemente bestimmbarer, definierter Druck auf die Bruchenden aufgebracht werden, was zu einem verbesserten Zusammenwachsen der Bruchenden beiträgt. Da die Spannelemente ferner je nach Bedarf in zu Ketten oder komplexeren, beispielsweise netzartigen, Strukturen zusammengefügt werden können und über die durch die komplementäre Verzahnung an den Aufnahmen geschaffene Rastfunktion präzise ausgerichtet werden können, ist es insbesondere möglich, selbst schwierigste Brüche, beispielsweise Trümmerfrakturen oder dergleichen, zu fixieren, was mit herkömmlichen Fixierungsvorrichtungen bisher nicht möglich war. Die Größe der einzelnen Spannelemente bestimmt sich dabei je nach Anwendungsgebiet. So werden beispielsweise im Bereich der Wirbelsäulenchirurige größere Spannelemente verwendet, als im Bereich der Gesichtschirurige. Analog können auch die Ketten je nach Anwendungsgebiet länger oder kürzer ausfallen.

Die erfindungsgemäße Fixierungsvorrichtung kann dabei vorteilhaft auch noch während der Operation selbst ausgerichtet werden, so dass der behandelnde Chirurg aufgrund des modularen Aufbaus aus Spannelementen und Hülsenelementen kurzfristig beispielsweise die Position der Fixierungsvorrichtung und/oder die Ausrichtung einzelner Spannelemente zueinander anpassen kann, sollte dies aufgrund des Bruches notwendig sein. Beispielsweise kann der Chirurg beim "Richten" des Bruchs die Winkellage der Spannelemente so verändern, dass die Bruchenden sauber aneinander zu liegen kommen. Da durch die Verzahnung der Aufnahmen die vorstehend beschriebene Rastfunktion geschaffen wird, bleiben die ausgerichteten Spannelemente in der gewählten Winkellage und die Fixierungsvorrichtung kann dann am Knochen verschraubt werden, ohne das es zu einer "Verschiebung" der Spannelemente kommt. Dies führt zu einer besseren Ausrichtung und Fixierung der Bruchenden, was wiederum dazu beiträgt, dass der Bruch ohne Kallusbildung ausheilt.

Darüber hinaus besteht die erfindungsgemäße Fixierungsvorrichtung aus sehr wenigen unterschiedlichen Einzelteilen. Die Fixierungsvorrichtung ist daher einfach und kostengünstig herstellbar. Da die dabei verwendeten Spannelemente überdies spiegelsymmetrisch entlang ihrer Längsachse ausgebildet sind, ist es möglich, nahezu unendlich lange Ketten oder dergleichen aus einer Vielzahl identischer Bauteile, d.h. Spannelemente, zu erstellen. Die Zahl der notwendigen unterschiedlichen Teile wird somit signifikant verringert während gleichzeitig der Anwendungsbereich kompressionserzeugender Fixierungen von Knochenbrüchen erweitert wird.

Gemäß einer Ausführungsform der erfindungsgemäßen Fixierungsvorrichtung sind die Hülsenelemente derart ausgebildet, dass sie entlang ihrer Axialrichtung, vorzugsweise federelastisch, längenveränderbar sind. Dabei sind die Hülsenelemente unverlierbar in die Aufnahmen einbringbar und die Winkellage der Spannelemente zueinander ist durch eine vermittels der an den einander zugewandten Aufnahmen ausgebildeten Verzahnung realisierte Rastfunktion bis zum Einbringen der Befestigungsmittel veränderbar.

Die längenveränderliche Ausgestaltung der Hülsenelemente ermöglicht in vorteilhafter Weise, dass selbst in zusammengebautem Zustand der Spannelemente mit den in die Aufnahmen derselben eingesetzten Hülsenelemente die relative Position der Spannelemente, also deren Winkellage zueinander, weiterhin verändert werden kann. Dadurch kann der behandelnde Arzt nach der Ausrichtung der Bruchenden die Winkellage der Spannelemente noch soweit anpassen, wie dies für eine optimale Behandlung des Bruchs notwendig ist, um die eingangs genannte Kallusbildung, d.h. die schwielenartige Verdickung der Bruchenden aus überwachsendem Knochengewebe, bei der Heilung des Bruchs zu vermeiden. Durch das Eindrehen der in den Aufnahmen, genauer gesagt, den Hülsenelementen, gehaltenen Schrauben in den Knochen werden die Hülsenelemente zunächst in Längsrichtung zusammengedrückt, dann gestaucht und zuletzt gegebenenfalls schwach gequetscht, wodurch die relative Beweglichkeit der Spannelemente zueinander durch das feste Ineinandergreifen der an den Aufnahmen ausgebildeten Zähne verhindert wird. Die vorher eingestellte Winkellage der Spannelemente zueinander wird damit fixiert und die aus mehreren Spannelementen, Hülsenelementen, etc. bestehende Fixierungsvorrichtung wird dann, durch Verschrauben sämtlicher Aufnahmen am Knochen vermittels geeigneter Schrauben, befestigt, um den Heilungsverlauf optimal zu unterstützen.

Durch die unverlierbar in die Aufnahmen einfügbaren Hülsenelemente ist es überdies vorteilhaft möglich, dass nicht erst unmittelbar vor der OP ein OP-Pfleger nach Maßgabe durch den behandelnden Arzt, oder gar der Chirurg selbst, die notwendige Anzahl von Einzelteilen grob zusammensetzt und diese dann "am Körper montiert". Vielmehr ist es möglich, dass der behandelnde Arzt und/oder das behandelnde Krankenhaus vorkonfektionierte "Systeme", also Fixiervorrichtungen für Knochenfrakturen, bezieht, die aus unterschiedlich langen, zu Ketten oder dergleichen zusammengefügten Spannelementen, Hülsenelementen und dergleichen besteht. Beispielsweise können derlei vorkonfektionierte Fixiervorrichtungen für Knochenfrakturen als 2er, 3er, 4er oder 5er Verbund von Spannelementen und Hülsenelementen, etc. ausgebildet sein, die dann je nach Bruchart und zu behandelndem Knochen kurz vor der OP ausgewählt und im Rahmen der OP-Vorbereitung bereitgestellt werden.

Gemäß einer weiteren Ausführungsform der erfindungsgemäßen Fixierungsvorrichtung ist die zur Oberseite und/oder zur Unterseite eines jeden Spannelements gerichtete Fläche der Aufnahme, bzw. deren zum Aufnahmemittelpunkt gerichteter Innenwandbereich, konkav ausgebildet.

Die konkave Ausgestaltung der zur Oberseite und/oder zur Unterseite eines jeden Spannelements gerichtete Fläche der Aufnahme bringt den Vorteil, dass die zur Fixierung der Fixierungsvorrichtung für Knochenfrakturen verwendeten Schrauben so eingedreht werden können, dass die Oberseite des Schraubenkopfs mit der Werkzeugaufnahme idealerweise bündig mit der Aufnahme abschließt, so dass es keine überstehenden Kanten gibt, die das anliegende Gewebe beeinträchtigen könnten.

Gemäß einer anderen Ausführungsform der erfindungsgemäßen Fixierungsvorrichtung ist die an einer der Kontaktflächen der einander zugewandten Aufnahmen ausgebildete Verzahnung komplementär zu der an der anderen der Kontaktflächen der einander zugewandten Aufnahmen ausgebildeten Verzahnung.

Durch die Ausbildung einer komplementären Verzahnung an den jeweils in gegenseitige Anlage bringbaren Flächen der Aufnahmen der Spannelemente ist es möglich, eine genaue Rasterung und Steuerbarkeit bzw. Einstellbarkeit der Winkellage der Spannelemente zueinander sicherzustellen. Insbesondere kann es nämlich, wenn die Verzahnung der Aufnahmen nicht komplementär ausgebildet ist, vorkommen, dass die Spannelemente nicht in einer geraden Linie oder der gewünschten bzw. benötigten Winkellage zueinander angeordnet werden können. Aus diesem Grund ist erfindungsgemäß vorgesehen, dass die an den Kontaktflächen ausgebildete Verzahnung der jeweils miteinander in Anlage bringbaren Aufnahmen komplementär ausgebildet ist, so dass eine exakte Einstellung der Winkellage der Spannelemente zueinander möglich ist.

Gemäß einer Ausführungsform der erfindungsgemäßen Fixierungsvorrichtung sind die Befestigungsmittel Corticalisschrauben und der Schraubenkopf einer jeden Corticalisschraube ist, an dessen dem Gewinde zugewandter Unterseite, vorzugsweise konisch oder ballig ausgestaltet, so dass die zur Aufnahme weisende Fläche des Schraubenkopfs komplementär zu der jeweils dem Schraubenkopf zugewandten Fläche der Aufnahme ist.

Durch die Verwendung von im medizinischen Bereich üblicherweise zu Anwendung kommenden Corticalisschrauben ist es im Prinzip nicht nötig, neue Schrauben vor dem Einsatz im tierischen oder menschlichen Körper durch kostenintensive Maßnahmen auf ihre diesbezügliche Eignung zu prüfen und durch die dafür zuständigen Gremien freigeben zu lassen. Analog zur konkaven Ausgestaltung der zur Oberseite und/oder zur Unterseite eines jeden Spannelements gerichtete Fläche der Aufnahme bringt die konische oder ballige Ausgestaltung des Schraubenkopfs der Corticalisschrauben ferner den Vorteil, dass die zur Fixierung der Fixierungsvorrichtung für Knochenfrakturen verwendeten Schrauben so eingedreht werden können, dass die Oberseite des Schraubenkopfs mit der Werkzeugaufnahme idealerweise bündig mit der Aufnahme abschließt, so dass es keine überstehenden Kanten gibt, die das anliegende Gewebe beeinträchtigen könnten.

Die wie vorstehend beschrieben ausgebildeten Spannelemente lassen sich, in Verbindung mit den in die daran ausgebildeten Aufnahmen eingesetzten Hülsenelemente zu nahezu unendlich langen Ketten verbinden. Um diese Ketten stabil und zuverlässig, sozusagen "versatz- und wackelfrei" am Knochen befestigen zu können, ist es jedoch nötig, dass diejenigen Abschnitte der Spannelemente, welche die Schrauben halten, möglichst vollflächig auf dem Knochen zu liegen kommen. Zudem kann die zur Fixierung verwendete Corticalisschraube nur bis zu einer gewissen Tiefe in den Knochen eingeschraubt werden.

Die erfindungsgemäße Fixierungsvorrichtung kann gemäß einer weiteren Ausführungsform ferner zumindest ein Abschlußelement aufweisen, das ringförmig ausgestaltet ist und eine mit einer Verzahnung ausgestaltete Kontaktfläche hat, wobei das Abschlußelement ferner eine einstückig damit ausgebildete Hülse hat, die derart durch eine der Aufnahmen eines Spannelements einbringbar ist, dass die Verzahnung des Abschlußelements komplementär mit der an der Aufnahme ausgebildeten Verzahnung in Eingriff gelangt und das Spannelement durch Einbringen des Befestigungsmittels durch die in der Aufnahme des Spannelements aufgenommene Hülse des Abschlußelements am Knochen fixiert werden kann.

Durch dieses Abschlußelement, das einfach mit der Aufnahme eines Spannelements am jeweiligen Ende der Kette in Eingriff gebracht werden kann, kann in vorteilhafter Weise sichergestellt werden, dass die Corticalisschraube ausreichend geführt und nicht zu tief in den Knochen geschraubt wird, während gleichzeitig eine stabile Auflagefläche des "Kettenendes" am Knochen geschaffen werden kann.

Gemäß einer weiteren anderen Ausführungsform der erfindungsgemäßen Fixierungsvorrichtung kann zumindest eines der Spannelemente anstelle einer Aufnahme an einem Ende in Längsrichtung der Spannelemente, eine als Abschlußelement dienende Ringaufnahme aufweisen, in welche ein Hülsenelement und/oder ein Befestigungsmittel zur Fixierung am Knochen einbringbar ist.

Diese alternative Ausgestaltung für das Abschlußelement verringert die Zahl der zur Anwendung kommenden unterschiedlichen Teile und erzielt dabei in gleicher Weise die vorstehend in Zusammenhang mit dem separat ausgebildeten Abschlußelement erzielbaren Vorteile.

Gemäß einer anderen Ausführungsform der erfindungsgemäßen Fixierungsvorrichtung ist zumindest eines der Spannelemente mehrschenklig, vorzugsweise Y-förmig, einstückig um ein zentrales ringförmiges Aufnahmeelement ausgebildet ist, und ein jedes Schenkelende eines solchen mehrschenkligen Spannelements weist eine einstückig daran anschließende Aufnahme auf, an deren zur gedachten Mittellinie eines jeden Spannelements in Längsrichtung weisender Kontaktfläche eine Verzahnung ausgebildet ist.

Durch die Verwendung eines derart "komplex" ausgebildeten, mehrschenkligen Spannelements mit mehreren (beispielsweise drei, vier oder mehr) Schenkeln lassen sich komplexe Anordnungen aus miteinander verknüpften Spannelementen erstellen. Hierbei können in besonders bevorzugter Weise Mehrfachkombinationen aus den vorstehend beschriebenen "einfachen" Spannelementen und "komplexen" mehrschenkligen Spannelementen (z.B. ein Y-Verbund aus einem mehrschenkligen Spannelement und mehreren einfachen Spannelementen oder ein Sternverbund aus mehreren mehrschenkligen Spannelementen sowie mehreren einfachen Spannelementen, bis hin zu netzartigen Strukturen etc.) zur Anwendung kommen. Dies ist insbesondere von Vorteil, wenn aufgrund eines komplizierten Bruchs, beispielsweise im Bereich des Handgelenks, eine Mehrfachverspannung von Bruchenden und/oder Knochen für eine optimale Heilung des Bruchs notwendig ist.

Gemäß einer Ausführungsform der erfindungsgemäßen Fixierungsvorrichtung umfasst zumindest eines der Spannelemente anstelle einer der, oder beider, Aufnahmen, an zumindest einem Ende in Längsrichtung der Spannelemente, eine als Zahnscheibe oder dergleichen ausgestaltete, ringförmige Aufnahmescheibe, die bei der Verknüpfung von Spannelementen, Hülsenelementen und/oder Befestigungsmitteln zwischen die ringförmig ausgebildeten Aufnahmen anderer Spannelemente einfügbar ist, zur Ausbildung einer Y-förmigen, sternförmigen oder sonstigen Kombination von Spannelementen, Hülsenelementen und/oder Befestigungsmitteln.

Analog zu dem vorstehend beschriebenen, mehrschenkligen Spannelement ist es mittels des Spannelements mit einer als Zahnscheibe oder dergleichen ausgebildeten Aufnahmescheibe möglich, komplexe Anordnungen von mehrfach verknüpften Spannelementen zu erstellen. Die Ausgestaltung einzelner Spannelemente mit der als Zahnscheibe oder dergleichen ausgebildeten Aufnahmescheibe, die eine komplementär zu der Verzahnung der sonst an den Spannelementen vorgesehenen Aufnahmen ausgebildete Verzahnung aufweist, bringt überdies den Vorteil, dass die einzelnen Spannelemente aufgrund dieser "Zahnscheibe" statt in durch das mehrschenklige Spannelement vorgegebenen Winkellagen in willkürlich gewählten Winkellagen zueinander zu komplexen Formen zusammengefügt werden können. Hierdurch wird der Einsatzbereich der erfindungsgemäßen Fixierungsvorrichtung, bei welcher derlei Spannelemente zum Einsatz kommen, noch weiter ausgedehnt.

Weitere Ausführungsformen, Merkmale und Vorteile der Erfindung werden anhand der nachfolgenden Beschreibung von Ausführungsformen unter Bezugnahme auf die Figuren ersichtlich. Dabei zeigt:
- Fig. 1: eine perspektivische Ansicht einer beispielhaften Ausführungsform einer erfindungsgemäßen Fixierungsvorrichtung in loser Anordnung;
- Fig. 2: eine perspektivische Ansicht der Ausführungsform der Fixierungsvorrichtung aus Fig. 1 mit ineinander verzahnten Spannelementen;
- Fig. 3: eine Detailansicht einer beispielhaften Ausführungsform eines beispielhaften Spannelements, das bei der erfindungsgemäßen Fixierungsvorrichtung Anwendung findet;
- Fig. 4: eine Detailansicht einer beispielhaften Ausführungsform eines beispielhaften Hülsenelements, das bei der erfindungsgemäßen Fixierungsvorrichtung Anwendung findet;
- Fig. 5: eine Detailansicht einer beispielhaften Ausführungsform eines beispielhaften Abschlußelements, das bei der erfindungsgemäßen Fixierungsvorrichtung Anwendung findet;
- Fig. 6: eine perspektivische Ansicht einer beispielhaften Ausführungsform einer erfindungsgemäßen Fixierungsvorrichtung mit einem Abschlußelement;
- Fig. 7: eine Detailansicht einer weiteren beispielhaften Ausführungsform eines beispielhaften Abschlußelements, das bei der erfindungsgemäßen Fixierungsvorrichtung Anwendung findet;
- Fig. 8: eine Detailansicht einer weiteren beispielhaften Ausführungsform eines beispielhaften Spannelements, das bei der erfindungsgemäßen Fixierungsvorrichtung zur Anwendung kommen kann;

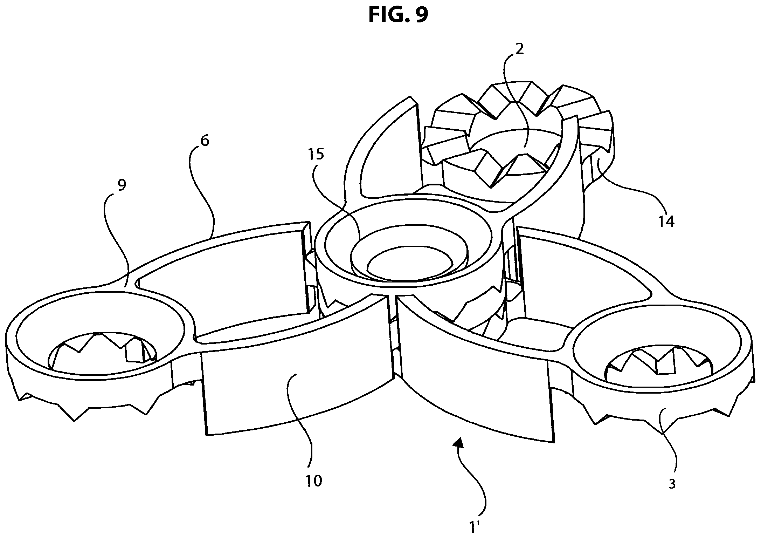

- Fig. 9: eine weitere Detailansicht einer anderen beispielhaften Ausführungsform eines beispielhaften Spannelements, das bei der erfindungsgemäßen Fixierungsvorrichtung zur Anwendung kommen kann; und
- Fig. 10: eine perspektivische Ansicht einer anderen beispielhaften Ausführungsform einer erfindungsgemäßen Fixierungsvorrichtung.

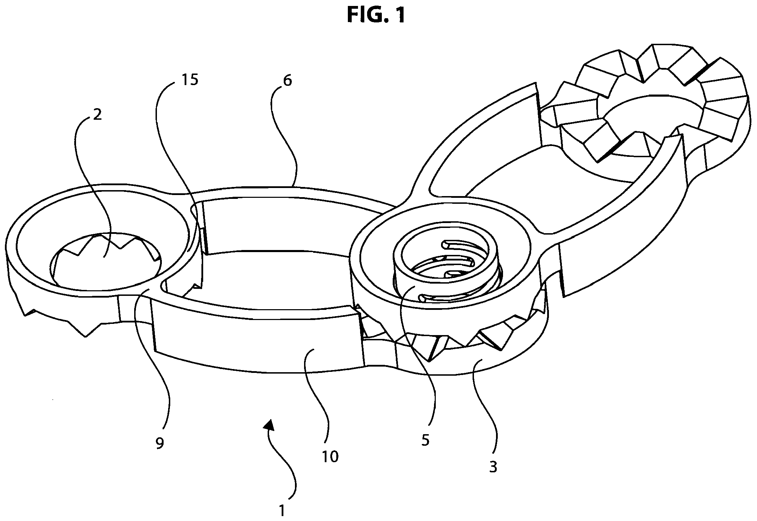

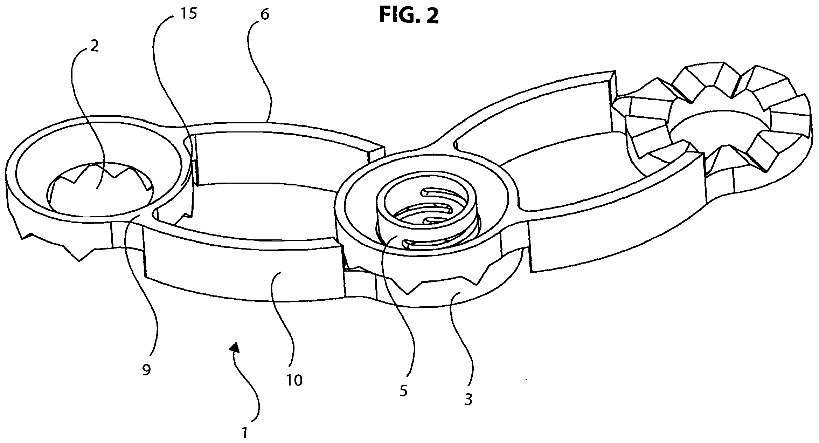

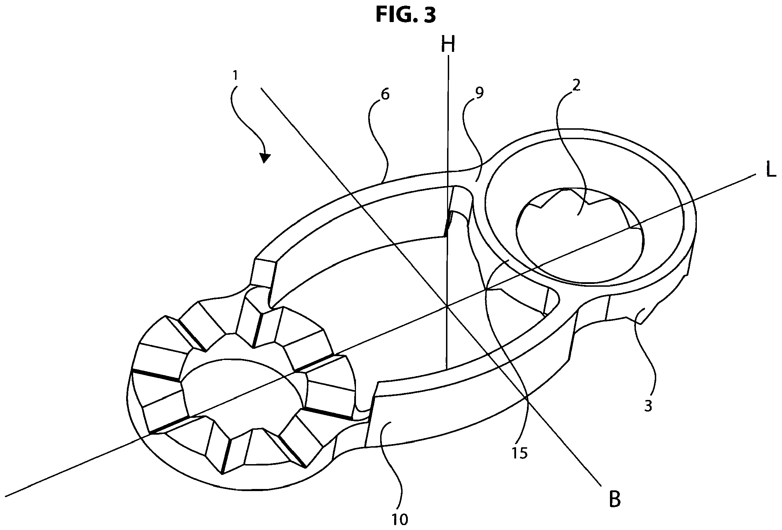

Nachfolgend wird eine bevorzugte Ausführungsform einer beispielhaften Fixierungsvorrichtung für Knochenfrakturen unter Bezugnahme auf die Figuren 1 bis 3 beschrieben. Hierbei zeigen die Figuren 1 und 2 eine perspektivische Ansicht einer Fixierungsvorrichtung für Knochenfrakturen, die aus zwei Spannelementen 1 gebildet ist, die vermittels eines Hülsenelements 5 miteinander verbunden sind. Fig. 3 zeigt ein bei dieser Ausführungsform zur Anwendung kommendes Spannelement 1 im Detail.

Es sei angemerkt, dass in Fig. 1 zwei Spannelemente 1 vermittels eines Hülsenelements 5 "lose" (also winkelveränderbar) miteinander verbunden sind, um das Grundprinzip der vorliegenden Erfindung zu verdeutlichen. Tatsächlich aber wird die Anzahl der für den zu behandelnden Bruch benötigten Spannelemente 1, beispielsweise drei, vier oder mehr Spannelemente 1, bedarfsgerecht bestimmt, und die benötigten Spannelemente 1 werden derart angeordnet, dass die jeweils an den Spannelementen 1 ausgebildeten Aufnahmen 2 einander überlappen, wie in den Figuren angedeutet ist.

Durch die sich überlappenden Aufnahmen 2 ist jeweils ein Hülsenelement 5 eingebracht, das an seinen oberen und unteren Enden umgebogen bzw. verstemmt wird (hier nicht dargestellt), sodass es verliersicher in den überlappenden Aufnahmen 2 gehalten ist.

Jede der Aufnahmen 2 des Spannelements 1 weist an ihrer zur gedachten Mittellinie eines jeden Spannelements 1 in Längsrichtung L weisende Kontaktfläche eine Verzahnung 3 auf, vermittels der die einzelnen Spannelemente 1 in einer vorbestimmten Winkellage zueinander ausrichtbar sind.

Durch das einander überlappende Anordnen der Aufnahmen 2 der einzelnen Spannelemente 1 gelangt, wie in den Figuren 1 und 2 gezeigt ist, die Verzahnung 3 an den jeweils zur gedachten Mittellinie der Spannelemente 1 in Längsrichtung gerichteten Kontaktflächen wechselseitig in Kontakt. Die Verzahnung 3 der jeweils an den in Längsrichtung des Spannelements 1 voneinander beabstandeten, umgekehrt zueinander angeordneten Aufnahmen 2 ist dabei komplementär ausgebildet, sodass die jeweils zur Mittellinie weisende Verzahnung 3 der voneinander beabstandeten Aufnahmen 2 die gleiche Zahnanzahl aufweist.

Je nach gewünschter Einstellbarkeit der Winkel einzelner Spannelemente 1 zueinander kann die Verzahnung 3 dabei mit mehr oder weniger Zähnen ausgebildet werden. Das bedeutet, je mehr Zähne die Verzahnung 3 aufweist, desto feiner ist die Einstellbarkeit der Winkel der Spannelemente 1 zueinander. In der hier dargestellten Ausführungsform ist die Verzahnung 3 als Zick-Zack-Muster ausgebildet. Die Verzahnung 3 kann jedoch auch als Wellenprofil, Rautenprofil oder in jeder anderen denkbaren, formschlüssigen Weise ausgebildet sein, so lange sich damit die Winkellage der Spannelemente 1 zueinander entsprechend einstellen und fixieren lässt.

Fig. 2 zeigt den Zustand, bei welchem die Spannelemente 1 in einem gewünschten Winkel ausgerichtet sind und durch das Ineinandergreifen der an den Aufnahmen 2 ausgebildeten Verzahnung 3 der einzelnen Spannelemente 1 in ihrer Position und Winkellage festgelegt sind.

Das durch die einander überlappenden Aufnahmen 2 der einzelnen Spannelemente 1 unverlierbar eingefügte Hülsenelement 5 fügt diese zu einer Kette zusammen, bei welcher die einzelnen Spannelemente 1 die Kettenglieder bilden und von dem Hülsenelement 5 relativ zueinander um das Hülsenelement 5 drehbar zusammengehalten werden.

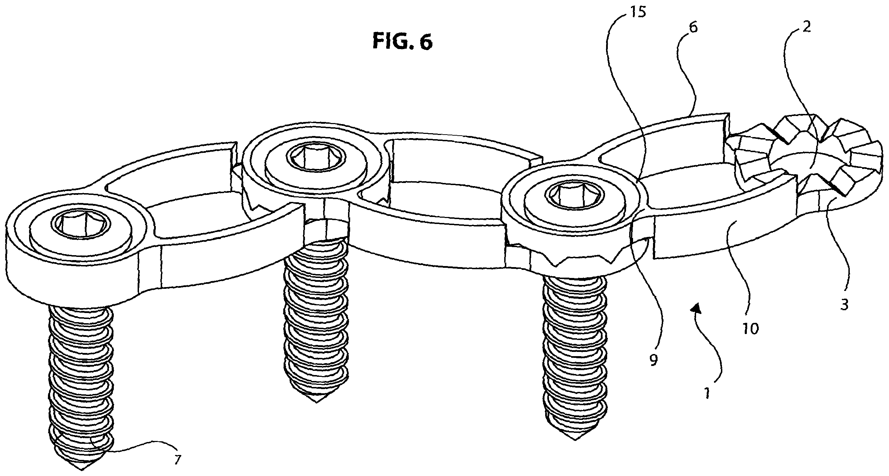

Das bedeutet, ein Hülsenelement 5 verbindet zwei Spannelemente 1 derart miteinander, dass die Zähne der an den Aufnahmen 2 ausgebildeten Verzahnung 3 dabei noch nicht oder nur leicht ineinander eingreifen und eine Rastfunktion bieten. Hierdurch kann die Winkellage der Spannelemente 1 zueinander eingestellt werden, wobei die gewählte Winkellage aufgrund der Rastfunktion beibehalten wird, auch wenn die Spannelemente 1 nicht mehr gehalten werden. Wenn eine (hier nicht abgebildete) Schraube 7 durch das in der Aufnahme 2 steckende Hülsenelement 5 geschraubt wird, wird die Winkellage, wie beispielsweise in Fig. 6 gezeigt, fixiert und ein weiteres Verdrehen der Spannelemente 1 relativ zueinander ist nicht möglich.

Um die vorstehend beschriebene Verdrehbarkeit der einzelnen Spannelemente 1 um das Hülsenelement 5 zu ermöglichen, ist das Hülsenelement 5 beispielsweise entsprechend lang ausgebildet, sodass es nach dem Einfügen in die Aufnahmen 2 und Verstemmen der Enden in Längsrichtung noch ausreichend "Bewegungsspielraum" für die miteinander verbundenen Spannelemente 1 für eine Drehung der Spannelemente 1 und der damit verbundenen Auf- und Ab-Bewegung entlang der Zahnflanken der Verzahnung 3 bietet.

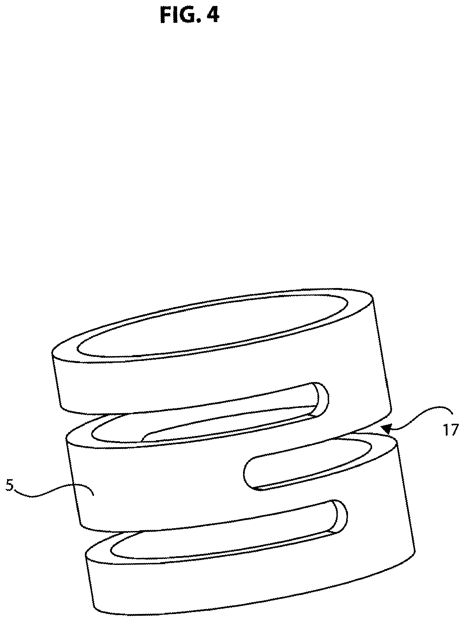

Bevorzugt ist das Hülsenelement 5 jedoch federelastisch entlang seiner Axialrichtung ausgebildet, sodass sich das Hülsenelement (auch als Federhülse bezeichnet) 5 beim Verdrehen der Spannelemente 1 relativ zueinander zur Einstellung des Winkel in Längsrichtung "strecken bzw. ausdehnen" kann, wodurch eine nachträgliche Anpassung des Winkels der Spannelemente 1 zueinander entlang der Rasterung der Verzahnung 3 an den Aufnahmen 2 möglich ist. Ein Beispiel eines derartig federelastischen Hülsenelements 5 ist in Fig. 4 abgebildet.

Das Hülsenelement 5 ist in der dort abgebildeten Ausführungsform im wesentlichen als kreisrunde Hülse ausgebildet, deren Wandung abwechselnd durch kreissegmentartige Ausschnitte 17 unterbrochen ist, um die federelastischen Eigenschaften zu schaffen. Alternativ zu der hier dargestellten Ausführungsform kann das Hülsenelement 5 auch ähnlich einer Spiralfeder ausgestaltet sein.

Sobald eine Schraube durch das Hülsenelement 5 geschraubt wird, um die zueinander ausgerichteten Spannelemente 1 zur Fixierung der Knochenfraktur am Knochen zu befestigen, wird das Hülsenelement 5 zunächst zusammengedrückt und dann gestaucht, je nach Eindrehkraft unter Umständen auch leicht gequetscht. Durch das Einschrauben der Schraube in den Knochen durch die in der Aufnahme 2 gehaltene Federhülse und das daraus resultierende ineinander greifen der Zähne der Verzahnung 3 der an den einzelnen Spannelementen 1 ausgebildeten Aufnahmen 2 wird der Winkel der Spannelemente 1 relativ zueinander unveränderlich fixiert.

Die Anordnung der einzelnen Spannelemente 1 zu einer Kette, in welcher die durch das Hülsenelement 5 jeweils verbundenen Spannelemente 1 die Kettenglieder bilden, kann dabei im Rahmen der OP-Vorbereitung durch entsprechend geschulte OP-Pfleger nach Vorgabe des behandelnden Arztes erfolgen.

Alternativ ist es jedoch auch denkbar, dass der behandelnde Arzt oder das behandelnde Krankenhaus vorgefertigte "Ketten" in einer sterilen, OP-geeigneten Umverpackung bezieht, wobei diese Ketten durch Kombinationen aus zwei, drei, vier oder mehr Spannelementen 1 mit entsprechend in die Aufnahmen 2 eingesetzten Hülsenelementen 5 derart vorgefertigt sind, dass die Hülsenelemente 5 bereits, durch das Verstemmen ihrer Enden in Längsrichtung, verliersicher in den einander überlappenden Aufnahmen 2 gehalten sind, gleichwohl aber eine Einstellung der Winkellage der Spannelemente 1 zueinander, wie vorstehend erläutert, noch erlauben. Idealerweise ist hier dann auch der gleichzeitige Bezug der entsprechend auf die jeweilige "Spannelement-Kette" abgestimmten Schrauben (z.B. im medizinischen Bereich häufig eingesetzte Corticalisschrauben sowie andere, speziell für das modulare Knochenplattensystem hergestellte Schrauben, aber auch Nägel oder Dübelsysteme) möglich.

Fig. 3 zeigt ein Spannelement 1, das bei der erfindungsgemäßen Fixierungsvorrichtung zu Anwendung kommt, im Detail.

Wie in Fig. 3 gezeigt ist, ist das Spannelement 1 oval oder ellipsenförmig ausgebildet und hat einen Konturkörper 6, der in Draufsicht hohl ist. Dieser Konturkörper 6 hat eine umlaufende Wandung mit zwei einander in Breitenrichtung B des Spannelements 1 gegenüberliegenden Seitenflanken 10, die, in Draufsicht betrachtet, von einem gedachten Mittelpunkt des Spannelements 1 bogenförmig nach außen gerichtet sind. Die Seitenflanken 10 gehen nahtlos in Y-förmige Verbindungsknoten 9 über, die in einstückig angeformte, in Draufsicht betrachtet zu dem gedachten Mittelpunkt des Spannelements 1 bogenförmig nach innen gerichtete, Aufnahmeabschnitte 15 auslaufen.

Wie aus den Figuren 1 bis 3 ersichtlich ist, sind die einzelnen Spannelemente 1 spiegelsymmetrisch zu ihrer Längsrichtung L ausgebildet, und die voneinander beabstandeten, vorzugsweise in Längsrichtung L des Spannelements 1 gegenüberliegenden, Aufnahmen 2 sind derart ausgestaltet, dass sich eine Aufnahme 2 der hier gezeigten Ausführungsform des Spannelements 1, vorzugsweise ausgehend von einer gedachten Mittellinie eines jeden Spannelements 1 in Längsrichtung L, in Richtung zur Außenkante des Konturkörpers 6 des Spannelements 1 in Höhenrichtung H des Konturkörpers 6 nach oben erstreckt und sich die andere Aufnahme 2, vorzugsweise ausgehend von der gedachten Mittellinie eines jeden Spannelements 1 in Längsrichtung L, in Richtung zur Außenkante des Konturkörpers 6 des Spannelements 1 in Höhenrichtung H des Konturkörpers 6 nach unten erstreckt.

Das Spannelement 1 hat ferner zwei voneinander beabstandete, sich vorzugsweise in Längsrichtung L des Spannelements 1 gegenüberliegende, ringförmige Aufnahmen 2, die zur Aufnahme der Hülsenelemente 5 und/oder der Befestigungsmittel 7 (hier nicht dargestellt) dienen, die jeweils durch die Aufnahmen 2 hindurchsteckbar sind. Die Aufnahmeabschnitte 15 sind integraler Bestandteil der ringförmige Aufnahmen 2, so dass diese einstückig mit dem Konturkörper 6 ausgestaltet sind und ein Hülsenelement 5 und/oder Befestigungsmittel 7 umschließen.

In der in Fig. 3 dargestellten Ausführungsform des Spannelements 1 sind die Aufnahmen 2 an deren Ober- bzw. Unterseite fluchtend mit den Seitenflanken 10 des Spannelements 1 ausgebildet.

Gleichwohl ist es nicht notwendig, dass die Aufnahmen von der gedachten Mittellinie der Spannelemente 1 in Längsrichtung bis an den oberen und/oder unteren Rand des Konturkörpers 6 reichen. So lange die Festigkeit der Aufnahme 2 gewährleistet werden kann, kann die Aufnahme 2 auch so ausgebildet sein, dass ihre Höhe nicht ganz bis an den oberen und/oder unteren Rand des Konturkörpers 6 reicht und beispielsweise eine Stufe am Übergang zwischen der Aufnahme 2 und den Seitenflanken 10 gebildet ist, oder ein fließender Übergang von der Aufnahme 2 zu den Seitenflanken 10 stattfindet. So ist es beispielsweise auch denkbar, dass die zur Ober- bzw. Unterseite des Konturkörpers 6 gerichtete Fläche der Aufnahme von ihrem in Längsrichtung des Spannelements 1 außen liegenden Rand hin zu den Seitenflanken 10 in einem vorgegebenen Winkel ansteigt.

Aufgrund der Verwendung im medizinischen Bereich wird das Spannelement 1 aus biokompatiblen Materialen hergestellt. Zudem ist ebenfalls ein Hybridaufbau möglich, bei dem die Wandung des Konturkörpers 6 im Bereich der Seitenflanken 10 aus einem Material mit der gewünschten elastischen Eigenschaft besteht und weitere Bereiche des Spannelements 1, wie z.B. die Aufnahmen 2, aus einem steifen oder resorbierbaren Material gebildet werden. Ein derartiger Hybridaufbau lässt sich beispielsweise durch die Auswahl eines entsprechenden Kompositprofils, das über den Querschnitt betrachtet aus Abschnitten verschiedener Werkstoffe zusammengesetzt ist, realisieren. Durch die elastische Ausbildung der Seitenflanken 10 ist es möglich, eine definierte Spannung in jedes Spannelement 1 einzubringen, wodurch eine kompressionserzeugende Fixierungen von Knochenbrüchen möglich wird.

Als Ausgangsmaterial für das Spannelement 1 kommen beispielsweise Metalle aus der Gruppe: X42CrMo15, X100CrMo17, X2CrNiMnMoNNb21-16-5-3, X20Cr13, X15Cr13, X30Cr13, X46Cr13, X17CrNi16-2, X14CrMoS17, X30CrMoN15-1, X65CrMo 17-3, X55CrMo14, X90CrMoV18, X50CrMoV15, X 38CrMo V15, G-X 20CrMo13, X39CrMo17-1, X40CrMoVN16-2, X105CrMo17, X20CrNiMoS13-1, X5CrNi18- 0, X8CrNiS18-9, X2CrNi19-11, X2CrNi18-9, X10CrNi18-8, X5CrNiMo17-12-2, X2CrNiMo17-12-2, X2CrNiMoN25-7-4, X2CrNiMoN17-13-3, X2CrNiMo17-12-3, X2CrNiMo18-14-3, X2CrNiMo18-15-3; X 2 CrNiMo 18 14 3, X13CrMnMoN18-14-3, X2CrNiMoN22136, X2CrNiMnMoNbN21-9-4-3, X4CrNiMnMo21-9-4, X105CrCoMo18-2, X6CrNiTi18-10, X5CrNiCuNb16-4, X3CrNiCuTiNb12-9, X3CrNiCuTiNb12-9, X7CrNiAl17-7, CoCr20Ni15Mo, G-CoCr29Mo, CoCr20W15Ni, Co-20Cr-15W-10Ni, CoCr28MoNi, CoNi35Cr20Mo10, Ti1, Ti2, Ti3, Ti4, Ti-5Al-2,5Fe, Ti-5Al-2,5Sn, Ti-6Al-4V, Ti-6Al-4V ELI, Ti-3Al-2,5V (Gr9), 99,5Ti, Ti-12Mo-6Zr-2Fe, Ti-13,4Al-29Nb, Ti-13Nb-13Zr, Ti-15Al, Ti-15Mo, Ti- 15Mo-5Zr-3Al, Ti-15Sn, Ti-15Zr-4Nb, Ti-15Zr-4Nb-4Ta, Ti-15Zr-4Nb-4Ta-0,2Pd, Ti-29Nb-13Ta-4,6Zr, Ti-30Nb-10Ta-5Zr, Ti-35,5Nb-1,5Ta-7,1Zr, Ti-35Zr-10Nb, Ti-45Nb, Ti-30Nb, Ti-30Ta, Ti-6Mn, Ti-5Zr-3Sn-5Mo-15Nb, Ti-3Al-8V-6Cr-4Zr-4Mo, Ti-6Al-2Nb-1Ta-0,8Mo, Ti-6Al-4Fe, Ti-6Al-4Nb, Ti-6Al-6Nb-1Ta, Ti-6Al-7Nb, Ti-6Al-4Zr-2Sn-2Mo, Ti-8,4Al-15,4Nb, Ti-8Al-7Nb, Ti-8Al-1Mo-1V, Ti-11Mo-6Zr-4Sn, in Betracht.

Ferner kommen Polymere aus der Gruppe: MBS, PMMI, MABS, CA, CTA, CAB, CAP, COC, PCT, PCTA, PCTG, EVA, EVAL, PTFE, ePTFE, PCTFE, PVDF, PVF, ETFE, ECTFE, FEP, PFA, LCP, PMMA, PMP, PHEMA, Polyamid 66, Polyamid 6, Polyamid 11 , Polyamid 2, PAEK, PEEK, PB, PC, PPC, PETP, PBT, MDPE, LDPE, HDPE, UHMWPE, LLDPE, PI, PAI, PEI, PIB, POM, PPO, PPE, PPS, PP, PS, PSU, PESU, PVC, PVC-P, PVC-U, ABS, SAN, TPE-U, TPE-A, TPE-E, PVDC, PVA, SI, PDMS, EPM, EP, UF, MF, PF, PUR, UP, PEBA, PHB, PLA, PLLA, PDLA, PDLLA, PGL, PGLA, PGLLA, PGDLLA, PGL-co-poly TMC, PGL-co-PCL, PDS, PVAL, PCL, Poly-TMC, PUR (linear), NiTi Superelastic, NiTi Shape Memory, in Betracht.

Des Weiteren kommen Keramiken aus der Gruppe: Al₂O₃ (Aluminiumoxid), Y-TZP (Zirkonoxidkeramik), AMC (Alumina Matrix Composite), HA (Hydroxilapatit), TCP (Tricalciumphosphat), Ceravital (Glaskeramik/Bioglas®), FZM/K (Zirkonoxid, teilstabilisiert), TZP-A (Zirkonoxidkeramik), ATZ (Alumina-toughened Zirconia), C799 (Aluminiumoxidkeramik), Schott 8625 (Transponderglas), in Betracht.

Darüber hinaus kommen Kombinationen hiervon in Betracht.

Hinsichtlich der Größe der einzelnen Spannelemente 1 und dem betreffenden Knochen gibt es grundsätzlich keine festgelegten Vorgaben. Vielmehr richtet sich die Größe der Spannelemente 1 nach dem Einsatzbereich, unterscheidet sich also mithin deutlich zwischen der Anwendung zur Fixierung von, zum Beispiel, Beinbrüchen und Gesichts- bzw. Schädelbrüchen oder dergleichen. Als grober Anhaltspunkt zur Größenauswahl ist beispielsweise eine proportionale Dimensionierung des Spannelements 1 zur dem verwendeten Schraubensystem denkbar. Aus der praktischen Medizintechnik kann dazu nährungsweise ein Zusammenhang zwischen dem Nenndurchmesser der Corticalis- bzw. Spongiosaschraube und dem Anwendungsbereich hergestellt werden, der in folgender Tabelle aufgeführt ist.

| Schraubensystem | Einsatzbereiche |
|---|---|
| HA 1,5 Corticalis | MGK-Chirurgie |
| HA 2,0 Corticalis | Cranio; Fußchirurgie; Handchirurgie |
| HA 2,5 Corticalis | Cranio; Fußchirurgie; Trauma |
| HA 3,5 Corticalis | Thorax; Unterer Rücken; Armchirurgie |
| HA 4,0 Corticalis | Thorax; Wirbelsäule; Hüft- und Beckenbereich |
| HA 4,5 Corticalis | Beinchirurgie; Rumpf; Fibula; Schulter |
| HA 5,0 Corticalis | Beinchirurgie; Tibia; Femur |
| HB 4,0 Spongiosa | abhängig vom Belastungsfall |
| HB 6,0 Spongiosa | abhängig vom Belastungsfall |

Die Wahl der Dimensionierung und Positionierung des Schraubensystems sowie des Spannelements 1 hängt zudem von der Frakturart (z.B. Querfraktur, Schrägfraktur) und dem Frakturort ab, wodurch sich verschiedene Belastungsfälle ergeben.

So kommen bei Schaftfrakturen zumeist Corticalissysteme zum Einsatz, da hier kein Spongiosaanteil vorhanden ist. Bei gelenknahen Frakturen kommen hingegen oftmals Spongiosasysteme zum Einsatz, da der Anteil der Spongiosa in diesem Bereich sehr hoch ist. Spongiosaschrauben weisen einen höheren Traganteil auf, da diese bei demselben Nenndurchmesser einen größeren Kerndurchmesser aufweisen. Bei Gelenkfrakturen, d.h. Frakturen mit Beteiligung der Gelenkfläche, werden in Abhängigkeit der lokalen anatomischen Bedingungen sowohl Cortikalissysteme als auch Spongiosasysteme verwendet. Ebenso können bei einer mehrfachen Fraktur beide Systeme eingesetzt werden.

Zudem können neben einer einfachen, d.h. monocorticalen Fixationsmöglichkeit auch bicorticale Systeme vorgesehen sein, bei denen die Schraube durch den Knochen hindurch an beiden corticalen Bereichen fixiert wird.

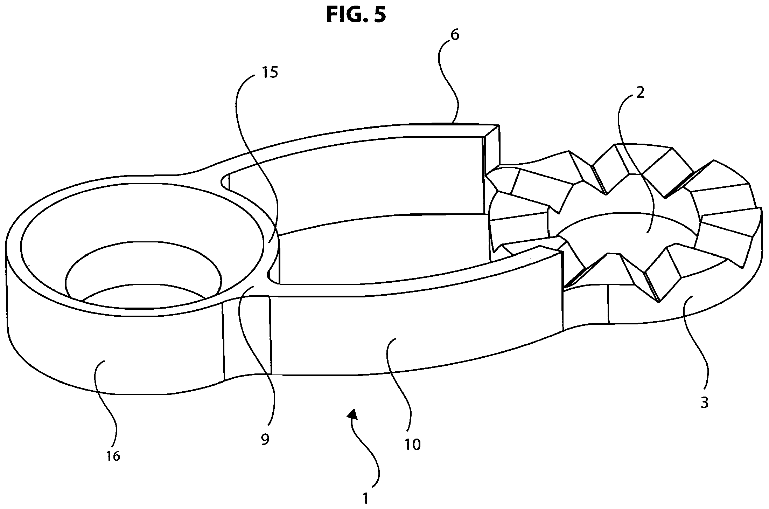

Wie in Fig. 6 gezeigt ist, sind an den Enden einer Spannelement-Kette als Abschlußelemente dienende Spannelemente 1 vorgesehen, die ein Fixieren der Enden der so zusammengesetzten Fixierungsvorrichtung mit entsprechenden Schrauben 7 am Knochen erlauben. Fig. 5 zeigt eine beispielhafte Ausgestaltung eines solchen Spannelements 1 mit Abschlußelement. Wie aus Fig. 5 ersichtlich ist, ist dieses Abschlußelement dabei weitestgehend wie ein herkömmliches Spannelement 1 aufgebaut, hat jedoch, an einem Ende in Längsrichtung, statt der die Verzahnung 3 aufweisenden Aufnahme 2, eine Ringaufnahme 16, in welche das Hülsenelement 5 und eine Schraube 7 zur Fixierung am Knochen einbringbar ist. Wie auch bei den mit der Verzahnung versehenen Aufnahmen 2 ist zumindest die dem Schraubenkopf zugewandet Kontaktfläche, bzw. der zum Aufnahmemittelpunkt gerichtete Innenwandbereich, der Ringaufnahme 17 konkav ausgebildet, sodass der Schraubenkopf möglichst bündig zur Oberkante der Aufnahme 2 (der Ringaufnahme 17) eingeschraubt werden kann und damit einer Irritation des umliegenden Gewebes vermeidet. Da bei den Abschlußelementen keine Verdrehbarkeit der einzelnen Spannelemente 1 zueinander gegeben sein muß, ist es auch möglich, die Schraube 7 ohne dazwischengefügte Hülse in die Ringaufnahme 17 einzubringen und das Spannelement 1 dann an den Knochen zu schrauben.

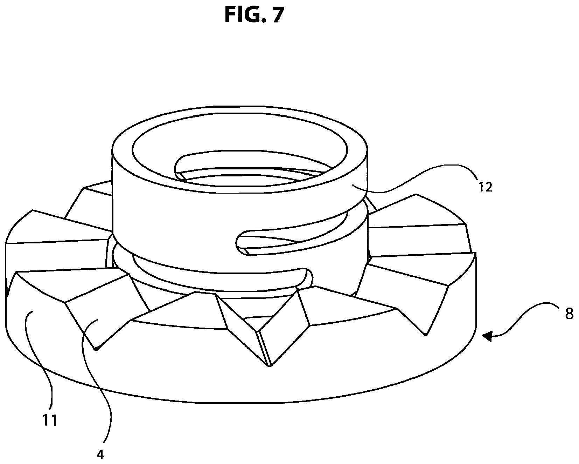

Eine alternative Ausgestaltung eines Abschlußelements 8 ist in Fig. 7 dargestellt. Dieses Abschlußelement 8 ist ringförmig ausgestaltet und hat eine mit einer Verzahnung 4 ausgestaltete Kontaktfläche 11 und eine einstückig damit vorgesehene Hülse 12, die durch eine der Aufnahmen 2 eines Spannelements 1 einbringbar ist, sodass die Verzahnung 4 des Abschlußelements 8 komplementär mit der an der Aufnahme 2 ausgebildeten Verzahnung 3 in Eingriff gelangt und das Spannelement 1 durch Einbringen einer Schraube 7 durch die in der Aufnahme 2 des Spannelements 1 aufgenommene Hülse 12 des Abschlußelements 8 am Knochen fixiert werden kann.

Derartige Abschlußelemente sind notwendig, um ein glattes Aufliegen der Enden der aus verschiedenen Spannelementen 1 und Hülsenelementen 5 zusammengefügten "Kette" auf dem Knochen zu gewährleisten.

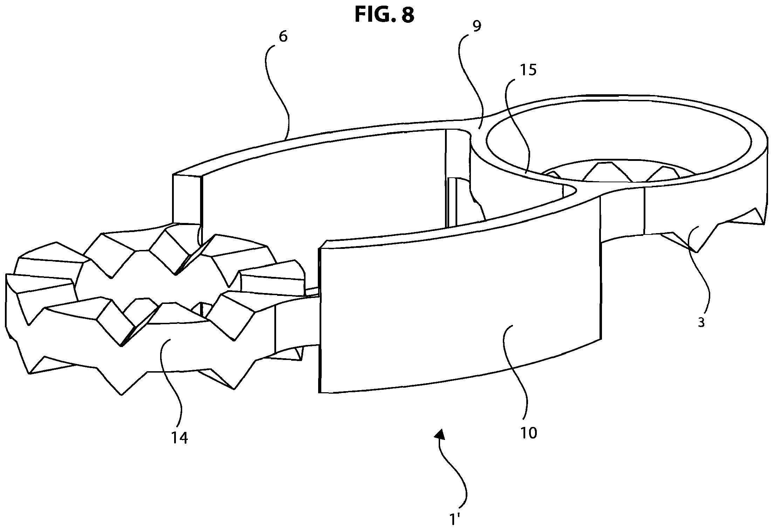

Anstelle von oder zusätzlich zu den vorstehend insbesondere in Zusammenhang mit den Figuren 1 bis 3 beschriebenen Spannelementen 1 kann auch ein in Fig. 8 gezeigtes Spannelement 1' zum Ausbilden der erfindungsgemäßen Fixierungsvorrichtung zur Anwendung kommen.

Dieses Spannelement 1' unterscheidet sich von dem vorstehend beschriebenen Spannelement 1 dadurch, dass anstelle einer der, oder beider, Aufnahmen 2, an zumindest einem Ende in Längsrichtung des Spannelementes 1' eine als Zahnscheibe oder dergleichen ausgestaltete, ringförmige Aufnahmescheibe 14 ausgebildet ist, die bei der Verknüpfung von Spannelementen, Hülsenelementen 5 und/oder Schrauben 7 zwischen die ringförmig ausgebildeten Aufnahmen 2 anderer Spannelemente 1 einfügbar ist, wie schematisch in Figur, 9 gezeigt ist. Durch die Verknüpfung eines oder mehrerer Spannelemente 1, 1' lassen such auf einfache Weise beispielsweise Y-förmige, sternförmige oder sonstige Kombination von Spannelementen, Hülsenelementen und/oder Schrauben realisieren.

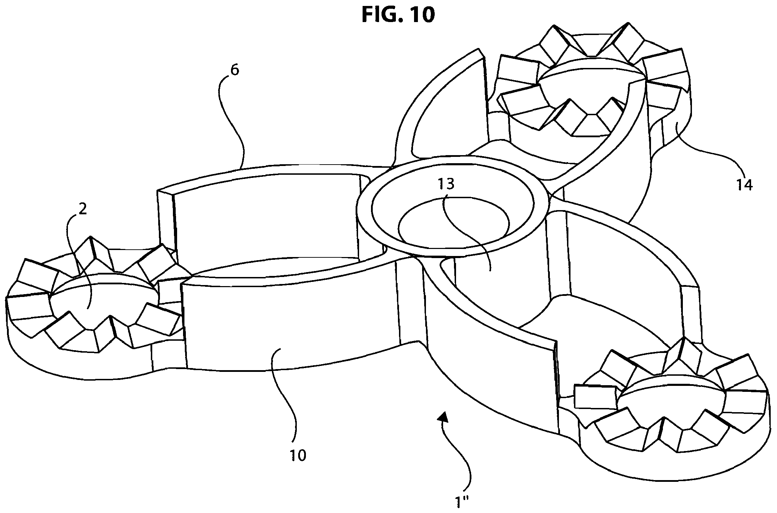

Eine weitere Ausgestaltung eines Spannelements ist in Fig. 10 dargestellt. Dieses Spannelement 1" unterscheidet sich von den vorstehend erläuterten Spannelementen dadurch, dass es anstelle der ovalen Gestalt eine mehrschenklige Gestalt hat. Insbesondere ist das in Fig. 10 abgebildete mehrschenklige Spannelement 1" Y-förmig einstückig um ein zentrales ringförmiges Aufnahmeelement 13 ausgebildet, und ein jedes Schenkelende dieses mehrschenkligen Spannelements 1" weist eine einstückig daran anschließende Aufnahme 2 auf, an deren zur gedachten Mittellinie des Spannelements 1" in Längsrichtung L weisender Kontaktfläche die Verzahnung 3 ausgebildet ist, vermittels der, wie vorstehend erläutert, eine Rastfunktion geschaffen wird, die ein Ausrichten der Spannelemente zueinander erlaubt und gleichzeitig, sobald die Spannelemente verschraubt werden, eine Fixierung der gewünschten Winkellage gewährleistet.

Obgleich in den Figuren nicht dargestellt, kann das mehrschenklige Spannelement 1" auch mit vier, fünf oder mehr Schenkeln ausgebildet sein. Die hier beschriebenen Spannelemente 1,1' und 1" sind willkürlich miteinander kombinierbar, so dass neben den vorstehend schon angesprochenen Ketten- oder Sternanordnungen jede denkbare Anordnung aus einer willkürlichen Kombination der einzelnen Spannelemente 1,1' und 1" geschaffen werden kann. Somit lassen sich beispielsweise auch komplexe "Netze" aus einer Kombination aller drei der hier dargestellten Spannelemente 1, 1' und 1" verwirklichen, wobei an den jeweiligen Enden der verknüpften Spannelemente 1,1' und 1" entweder die in Fig. 7 gezeigten, separaten Abschlußelemente 8 oder die in Fig. 6 gezeigten Spannelemente mit integrierter, als Abschlußelement dienender Ringaufnahme 16, zur Anwendung kommen.

Die vorliegende Erfindung schafft, wie vorstehend dargestellt, eine Fixierungsvorrichtung für Knochenfrakturen mit wenigstens zwei Spannelementen 1,1' und 1", wenigstens zwei Befestigungsmitteln 7, sowie wenigstens zwei Hülsenelementen 5. Ein jedes der Spannelemente 1,1' und 1" hat einen Konturkörper 6 und weist, an in Längsrichtung voneinander beabstandeten Enden, Aufnahmen 2 auf, die Hülsenelemente 5 und/oder Befestigungselemente 7 umschließen, so dass durch eine Verknüpfung mehrerer Spannelemente 1, Hülsenelemente 5 und/oder Befestigungsmittel 7 diese zu einer Kette oder dergleichen angeordnet werden können. Ein jedes Spannelement 1 ist punktsymmetrisch in Breitenrichtung bzw. spiegelsymmetrisch zur Längsrichtung ausgebildet, wobei sich die Aufnahmen 2 ausgehend von einer gedachten Mittellinie eines jeden Spannelements 1 in Längsrichtung L jeweils nach oben oder unten zur Außenkante des Konturkörpers 6 des Spannelements 1 in Höhenrichtung H erstrecken und an ihrer der gedachten Mittellinie zugewandten Kontaktfläche eine Verzahnung 3 aufweisen, wobei bei der Verbindung einzelner Spannelemente 1 miteinander diese, durch das miteinander in Eingriff bringen der an den einander zugewandten Kontaktflächen der Aufnahmen 2 ausgebildeten Verzahnung 3, in eine vorbestimmte Winkellage zueinander ausrichtbar sind.
1, 1', 1" ... Spannelement
2 ... ringförmige Aufnahme
3, 4 ... Verzahnung
5 ... Hülsenelement
6 ... Konturkörper
7 ... Befestigungsmittel
8 ... Abschlußelement
9 ... Verbindungsknoten
10 ... Seitenflanke
11 ... Kontaktfläche
12 ... Hülse
13 ... Aufhahmeelement
14 ... ringförmige Aufhahmescheibe
15 ... Aufnahmeabschnitt
16 ... Ringaufnahme
17 ... Ausschnitt
B ... Breitenrichtung
L ... Längsrichtung
H ... Höhenrichtung

## Patentansprüche

1. Fixierungsvorrichtung für Knochenfrakturen, aufweisend:
wenigstens zwei Spannelemente (1, 1', 1"), wenigstens zwei Befestigungsmittel (7), die, vorzugsweise im Bereich von Bruchenden, in den Knochen einbringbar sind, sowie wenigstens zwei Hülsenelemente (5), wobei
ein jedes Spannelement (1, 1', 1") aufweist:
• einen in Draufsicht hohlen, eine umlaufende Wandung aufweisenden, Konturkörper (6) mit zwei einander in Breitenrichtung (B) des Spannelements (1, 1', 1") gegenüberliegenden Seitenflanken (10), die, in Draufsicht betrachtet, von einem gedachten Mittelpunkt des Spannelements (1, 1', 1") bogenförmig nach außen gerichtet sind,
∘ wobei die Seitenflanken (10) nahtlos in Y-förmige Verbindungsknoten (9) übergehen, die in einstückig angeformte, in Draufsicht betrachtet zu dem gedachten Mittelpunkt des Spannelements (1, 1', 1") bogenförmig nach innen gerichtete, Aufnahmeabschnitte (15) auslaufen,
• wenigstens zwei voneinander beabstandete, sich vorzugsweise in Längsrichtung (L) des Spannelements (1, 1', 1") gegenüberliegende, ringförmige Aufnahmen (2), die zur Aufnahme der Hülsenelemente (5) und/oder der Befestigungsmittel (7) dienen, die jeweils durch die Aufnahmen (2) hindurchsteckbar sind,
∘ wobei die Aufnahmeabschnitte (15) integraler Bestandteil der ringförmige Aufnahmen (2) sind, so dass diese einstückig mit dem Konturkörper (6) ausgestaltet sind und ein Hülsenelement (5) und/oder Befestigungsmittel (7) umschließen, und wobei
durch eine Verknüpfung mehrerer Spannelemente (1, 1', 1"), Hülsenelemente (5) und/oder Befestigungsmittel (7) diese zu einer Kette angeordnet werden können;
wobei die umlaufende Wandung des Konturkörpers (6) eines jeden Spannelements (1, 1', 1") wenigstens abschnittsweise im Bereich der Seitenflanken (10) federelastisch verformbar ist,
wobei ein jedes Spannelement (1, 1', 1") spiegelsymmetrisch zur Längsrichtung (L) des Spannelements (1, 1', 1") ausgebildet ist und die voneinander, vorzugsweise in Längsrichtung (L) des Spannelements (1, 1', 1") gegenüberliegenden, beabstandeten Aufnahmen (2) derart ausgestaltet sind, dass
• sich eine Aufnahme (2), vorzugsweise ausgehend von einer gedachten Mittellinie eines jeden Spannelements (1, 1', 1") in Längsrichtung (L), in Richtung zur Außenkante des Konturkörpers (6) des Spannelements (1, 1', 1") in Höhenrichtung (H) des Konturkörpers (6) nach oben erstreckt, und
• sich die andere Aufnahme (2), vorzugsweise ausgehend von der gedachten Mittellinie eines jeden Spannelements (1, 1', 1") in Längsrichtung (L), in Richtung zur Außenkante des Konturkörpers (6) des Spannelements (1, 1', 1") in Höhenrichtung (H) des Konturkörpers (6) nach unten erstreckt,
wobei eine jede ringförmige Aufnahme (2) an ihrer zur gedachten Mittellinie eines jeden Spannelements (1, 1', 1") in Längsrichtung (L) weisende Kontaktfläche eine Verzahnung (3) aufweist,
wobei einzelne Spannelemente (1, 1', 1") derart miteinander verbunden werden können, dass die sich, vorzugsweise ausgehend von der gedachten Mittellinie eines jeden Spannelements (1, 1', 1") in Längsrichtung (L), in Richtung zur Außenkante des Konturkörpers (6) des Spannelements (1, 1', 1") in Höhenrichtung (H) des Konturkörpers (6) nach oben oder unten erstreckenden Aufnahmen (2) derart übereinander zu liegen kommen, dass die zur gedachten Mittellinie des Spannelements (1, 1', 1") weisenden Kontaktflächen der Aufnahmen (2) einander, im wesentlichen deckungsgleich, zugewandt sind,
und wobei, durch das miteinander in Eingriff bringen der an den jeweils zur gedachten Mittellinie eines jeden Spannelements (1, 1', 1") in Längsrichtung (L) weisenden, einander zugewandten Kontaktflächen der Aufnahmen (2) ausgebildeten Verzahnung (3), die Spannelemente (1, 1', 1") in einer vorbestimmten Winkellage zueinander ausrichtbar sind.

2. Fixierungsvorrichtung nach Anspruch 1, wobei die Hülsenelemente (5) derart ausgebildet sind, dass sie entlang ihrer Axialrichtung, vorzugsweise federelastisch, längenveränderbar sind.

3. Fixierungsvorrichtung nach Anspruch 1 oder 2, wobei die Hülsenelemente (5) unverlierbar in die Aufnahmen (2) einbringbar sind.

4. Fixierungsvorrichtung nach einem der Ansprüche 1 bis 3, wobei die Winkellage der Spannelemente (1, 1', 1") zueinander durch eine vermittels der an den einander zugewandten Aufnahmen (2) ausgebildeten Verzahnung (3) realisierte Rastfunktion bis zum Einbringen der Befestigungsmittel (7) veränderbar ist.

5. Fixierungsvorrichtung nach einem der Ansprüche 1 bis 4, wobei die zur Oberseite und/oder zur Unterseite eines jeden Spannelements (1, 1', 1") gerichtete Fläche der Aufnahme (2) konkav ausgebildet ist.

6. Fixierungsvorrichtung nach Anspruch 1, wobei die an einer der Kontaktflächen der einander zugewandten Aufnahmen (2) ausgebildete Verzahnung (3) komplementär zu der an der anderen der Kontaktflächen der einander zugewandten Aufnahmen (2) ausgebildeten Verzahnung (3) ist.

7. Fixierungsvorrichtung nach Anspruch 1, wobei die Befestigungsmittel Corticalisschrauben sind.

8. Fixierungsvorrichtung nach Anspruch 5, wobei der Schraubenkopf einer jeden Corticalisschraube konisch oder ballig ausgestaltet ist, so dass die zur Aufnahme (2) weisende Fläche des Schraubenkopfs komplementär zu der jeweils dem Schraubenkopf zugewandten Fläche der Aufnahme (2) ist.

9. Fixierungsvorrichtung nach einem der Ansprüche 1 bis 8, weiter aufweisend zumindest ein Abschlußelement (8), das ringförmig ausgestaltet ist und eine mit einer Verzahnung (4) ausgestaltete Kontaktfläche (11) hat, wobei das Abschlußelement (8) ferner eine einstückig damit ausgebildete Hülse (12) hat, die derart durch eine der Aufnahmen (2) eines Spannelements (1, 1', 1") einbringbar ist, dass die Verzahnung (4) des Abschlußelements (8) komplementär mit der an der Aufnahme (2) ausgebildeten Verzahnung (3) in Eingriff gelangt und das Spannelement (1, 1', 1") durch Einbringen des Befestigungsmittels (7) durch die in der Aufnahme (2) des Spannelements (1, 1', 1") aufgenommene Hülse (12) des Abschlußelements (8) am Knochen fixiert werden kann.

10. Fixierungsvorrichtung nach einem der Ansprüche 1 bis 8, wobei zumindest eines der Spannelemente (1, 1', 1") anstelle einer Aufnahmen (2) an einem Ende in Längsrichtung der Spannelemente (1, 1', 1"), eine als Abschlußelement dienende Ringaufnahme (16) aufweist, in welche ein Hülsenelement (5) und/oder ein Befestigungsmittel (7) zur Fixierung am Knochen einbringbar ist.

11. Fixierungsvorrichtung nach einem der Ansprüche 1 bis 8, wobei zumindest eines der Spannelemente (1, 1', 1") mehrschenklig, vorzugsweise Y-förmig, einstückig um ein zentrales ringförmiges Aufnahmeelement (13) ausgebildet ist, und ein jedes Schenkelende eines solchen mehrschenkligen Spannelements (1, 1', 1") eine einstückig daran anschließende Aufnahme (2) aufweist, an deren zur gedachten Mittellinie eines jeden Spannelements (1, 1', 1") in Längsrichtung (L) weisender Kontaktfläche eine Verzahnung (3) ausgebildet ist.

12. Fixierungsvorrichtung nach einem der Ansprüche 1 bis 8, wobei zumindest eines der Spannelemente (1, 1', 1") anstelle einer der, oder beider, Aufnahmen (2), an zumindest einem Ende in Längsrichtung der Spannelemente (1, 1', 1"), eine als Zahnscheibe oder dergleichen ausgestaltete, ringförmige Aufnahmescheibe (14) umfasst, die bei der Verknüpfung von Spannelementen (1, 1', 1"), Hülsenelementen (5) und/oder Befestigungsmitteln (7) zwischen die ringförmig ausgebildeten Aufnahmen (2) anderer Spannelemente (1, 1', 1") einfügbar ist, zur Ausbildung einer Y-förmigen, sternförmigen oder sonstigen Kombination von Spannelementen (1, 1', 1"), Hülsenelementen (5) und/oder Befestigungsmitteln (7).

## Claims

1. Fixing device for bone fractures, comprising:
at least two tensioning elements (1, 1', 1"), at least two fastening means (7) which, preferably in the region of ends of the fracture, can be introduced into the bone, and at last two sleeve elements (5), wherein
each tensioning element (1, 1', 1") has:
• a contoured body (6) which is hollow in plan view, has a circumferential wall and has two lateral flanks (10) which lie opposite one another in the width direction (B) of the tensioning element (1, 1', 1") and which, as seen in plan view, are directed in an arcuate manner outwards from an imaginary centre point of the tensioning element (1, 1', 1").
∘ wherein the lateral flanks (10) merge seamlessly into Y-shaped connection points (9) which run out into integrally formed receiving sections (15) which, as seen in plan view, are directed in an arcuate manner inwards to the imaginary centre point of the tensioning element (1, 1', 1"),
• at least two mutually spaced apart annular receivers (2) which lie opposite one another preferably in the longitudinal direction (L) of the tensioning element (1, 1', 1") and which serve to receive the sleeve elements (5) and/or the fastening means (7) which can each be inserted through the receivers (2),
∘ wherein the receiving sections (15) are an integral component of the annular receivers (2), so that they are configured in one piece with the contoured body (6) and enclose a sleeve element (5) and/or fastening means (7), and wherein
by linking a plurality of tensioning elements (1, 1', 1"), sleeve elements (5) and/or fastening means (7) they can be arranged into a chain;
wherein the circumferential wall of the contoured body (6) of each tensioning element (1, 1', 1") is deformable in a spring-elastic manner at least in sections in the region of the lateral flanks (10),
wherein each tensioning element (1, 1', 1") is formed with mirror-symmetry with respect to the longitudinal direction (L) of the tensioning element (1, 1', 1") and the mutually spaced part receivers (2) which lie opposite one another preferably in the longitudinal direction (L) of the tensioning element (1, 1', 1") are configured in such a manner that
• a receiver (2), preferably starting from an imaginary centre line of each tensioning element (1, 1', 1") in the longitudinal direction (L), extends in the direction of the outer edge of the contoured body (6) of the tensioning element (1, 1', 1") upwards in the height direction (H) of the contoured body (6), and
• the other receiver (2), preferably starting from the imaginary centre line of each tensioning element (1, 1', 1") in the longitudinal direction (L), extends in the direction of the outer edge of the contoured body (6) of the tensioning element (1, 1', 1") downwards in the height direction (H) of the contoured body (6),
wherein each annular receiver (2) has a toothing system (3) on its contact surface pointing towards the imaginary centre line of each tensioning element (1, 1', 1") in the longitudinal direction (L),
wherein individual tensioning elements (1, 1', 1") can be connected to one another such that the receivers (2), which extend, preferably starting from the imaginary centre line of each tensioning element (1, 1', 1") in the longitudinal direction (L), in the direction of the outer edge of the contoured body (6) of the tensioning element (1, 1', 1") upwards or downwards in the height direction (H) of the contoured body (6) come to lie one on top of the other such that the contact surfaces of the receivers (2) pointing towards the imaginary centre line of the tensioning element (1, 1', 1") face one another substantially congruently,
and wherein the tensioning elements (1, 1', 1") can be aligned with respect to one another in a predetermined angular position by the mutual engagement of the toothing system (3) formed on the mutually facing contact surfaces of the receivers (2) pointing in each case towards the imaginary centre line of each tensioning element (1, 1', 1") in the longitudinal direction (L).

2. Fixing device as claimed in claim 1, wherein the sleeve elements (5) are formed in such a manner that their length can be changed, preferably in a spring-elastic manner, in their axial direction.

3. Fixing device as claimed in claim 1 or 2, wherein the sleeve elements (5) can be captively introduced into the receivers (2).

4. Fixing device as claimed in any one of claims 1 to 3, wherein the angular position of the tensioning elements (1, 1', 1") with respect to one another can be changed by means of a latching function, which is implemented by the toothing system (3) formed on the mutually facing receivers (2), until the fastening means (7) are introduced.

5. Fixing device as claimed in any one of claims 1 to 4, wherein the surface of the receiver (2) directed to the upper side and/or to the lower side of each tensioning element (1, 1', 1") is concave.

6. Fixing device as claimed in claim 1, wherein the toothing system (3) formed on one of the contact surfaces of the mutually facing receivers (2) is complementary to the toothing system (3) formed on the other one of the contact surfaces of the mutually facing receivers (2).

7. Fixing device as claimed in claim 1, wherein the fastening means are cortical screws.

8. Fixing device as claimed in claim 5, wherein the screw head of each cortical screw is configured in a conical or spherical manner, so that the surface of the screw head pointing towards the receiver (2) is complementary to the surface of the receiver (2) facing the screw head in each case.

9. Fixing device as claimed in any one of claims 1 to 8, further comprising at least one termination element (8) which is configured in an annular manner and has a contact surface (11) configured with a toothing system (4), wherein the termination element (8) further has a sleeve (12) which is integrally formed therewith and which can be introduced through one of the receivers (2) of a tensioning element (1, 1', 1") such that the toothing system (4) of the termination element (8) moves into engagement in a complementary manner with the toothing system (3) formed on the receiver (2) and the tensioning element (1, 1', 1") can be fixed to the bone by introducing the fastening means (7) through the sleeve (12) of the termination element (8) received in the receiver (2) of the tensioning element (1, 1', 1").

10. Fixing device as claimed in any one of claims 1 to 8, wherein at least one of the tensioning elements (1, 1', 1") has, instead of a receiver (2) at an end in the longitudinal direction of the tensioning elements (1, 1', 1"), an annular receiver (16) which serves as a termination element and into which a sleeve element (5) and/or a fastening means (7) for fixing to the bone can be introduced.

11. Fixing device as claimed in any one of claims 1 to 8, wherein at least one of the tensioning elements (1, 1', 1") is formed with multiple limbs, preferably in a Y-shape, in one piece around a central annular receiving element (13), and each limb end of such a multiple-limbed tensioning element (1, 1', 1") has a receiver (2) integrally adjoining thereto, on whose contact surface pointing towards the imaginary centre line of each tensioning element (1, 1', 1") in the longitudinal direction (L) a toothing system (3) is formed.

12. Fixing device as claimed in any one of claims 1 to 8, wherein at least one of the tensioning elements (1, 1', 1") comprises, instead of one, or both, of the receivers (2), on at least one end in the longitudinal direction of the tensioning elements (1, 1', 1"), an annular receiving disk (14) which is configured as a toothed disk or the like and which, when linking tensioning elements (1, 1', 1"), sleeve elements (5) and/or fastening means (7), can be inserted between the annular receivers (2) of other tensioning elements (1, 1', 1"), to form a Y-shaped, star-shaped or other combination of tensioning elements (1, 1', 1"), sleeve elements (5) and/or fastening means (7).

## Revendications

1. Dispositif de fixation pour des fractures osseuses, comportant :
au moins deux éléments tendeurs (1, 1', 1"), au moins deux moyens de fixation (7), qui peuvent être placés dans les os, de préférence dans la zone des extrémités de fracture, ainsi qu'au moins deux éléments enveloppants (5), dans lequel
chaque élément tendeur (1, 1', 1") comporte :
• un corps de contour (6), creux en vue de dessus, comportant une paroi périphérique, avec deux flancs latéraux (10) qui sont à l'opposé l'un de l'autre dans le sens de la largeur (B) de l'élément tendeur (1, 1', 1") et qui, en vue de dessus, sont dirigés en forme d'arc vers l'extérieur à partir d'un centre imaginaire de l'élément tendeur (1, 1', 1"),
∘ dans lequel les flancs latéraux (10) se transforment sans coupure en noeuds de liaison (9) en forme de Y qui se terminent en tronçons de logement (15) formés d'un seul tenant et dirigés, en vue de dessus, en forme d'arc vers l'intérieur par rapport au centre imaginaire de l'élément tendeur (1, 1', 1"),
• au moins deux logements annulaires (2), distants l'un de l'autre, qui sont de préférence à l'opposé l'un de l'autre dans le sens de la longueur (L) de l'élément tendeur (1, 1', 1") et qui servent à loger les éléments enveloppants (5) et/ou les moyens de fixation (7), lesquels peuvent être introduits à chaque fois à travers les logements (2),
∘ dans lequel les tronçons de logement (15) font partie intégrante des logements annulaires (2) de telle sorte que ceux-ci sont conçus d'un seul tenant avec le corps de contour (6) et entourent un élément enveloppant (5) et/ou des moyens de fixation (7), et dans lequel par une combinaison de plusieurs éléments tendeurs (1, 1', 1"), éléments enveloppants (5) et/ou moyens de fixation (7), ceux-ci peuvent être agencés en une chaîne ;
dans lequel la paroi périphérique du corps de contour (6) de chaque élément tendeur (1, 1', 1") est élastiquement déformable au moins en partie dans la zone des flancs latéraux (10),
dans lequel chaque élément tendeur (1, 1', 1") est formé avec une symétrie par rapport au sens de la longueur (L) de l'élément tendeur (1, 1', 1") et les logements (2) distants l'un de l'autre et à l'opposé l'un de l'autre de préférence dans le sens de la longueur (L) de l'élément tendeur (1, 1', 1") sont conçus de telle sorte
• qu'un logement (2) s'étend vers le haut dans le sens de la hauteur (H) du corps de contour (6) de préférence à partir d'une ligne médiane imaginaire de chaque élément tendeur (1, 1', 1") dans le sens de la longueur (L) en direction de l'arête extérieure du corps de contour (6) de l'élément tendeur (1, 1', 1")
• et que l'autre logement (2) s'étend vers le bas dans le sens de la hauteur (H) du corps de contour (6) de préférence à partir de la ligne médiane imaginaire de chaque élément tendeur (1, 1', 1") dans le sens de la longueur (L) en direction de l'arête extérieure du corps de contour (6) de l'élément tendeur (1, 1', 1"),
dans lequel chaque logement annulaire (2) présente une denture (3) au niveau de sa surface de contact regardant vers la ligne médiane imaginaire de chaque élément tendeur (1, 1', 1") dans le sens de la longueur (L),
dans lequel des éléments tendeurs (1, 1', 1") individuels peuvent être reliés entre eux de telle sorte que les logements (2) s'étendant vers le haut ou le bas dans le sens de la hauteur (H) du corps de contour (6) de préférence à partir de la ligne médiane imaginaire de chaque élément tendeur (1, 1', 1") dans le sens de la longueur (L) en direction de l'arête extérieure du corps de contour (6) de l'élément tendeur (1, 1', 1") viennent s'appuyer l'un sur l'autre de telle sorte que les surfaces de contact des logements (2) qui regardent vers la ligne médiane imaginaire de chaque élément tendeur (1, 1', 1") sont proches l'une de l'autre de manière à se recouvrir globalement,
et dans lequel, par la mise en prise de la denture (3) formée au niveau des surfaces de contact des logements (2) proches l'une de l'autre et regardant à chaque fois vers la ligne médiane imaginaire de chaque élément tendeur (1, 1', 1") dans le sens de la longueur (L), les éléments tendeurs (1, 1', 1") sont orientables les uns par rapport aux autres dans une position angulaire prédéterminée.

2. Dispositif de fixation selon la revendication 1, dans lequel les éléments enveloppants (5) sont formés de telle sorte qu'ils sont variables en longueur, de préférence de manière élastique, le long de leur direction axiale.

3. Dispositif de fixation selon la revendication 1 ou 2, dans lequel les éléments enveloppants (5) peuvent être placés, imperdables, dans les logements (2).

4. Dispositif de fixation selon l'une quelconque des revendications 1 à 3, dans lequel la position angulaire des éléments tendeurs (1, 1', 1") les uns par rapport aux autres est modifiable par une fonction de crantage, réalisée au moyen de la denture (3) formée au niveau des logements (2) proches l'un de l'autre, jusqu'à la mise en place des moyens de fixation (7).

5. Dispositif de fixation selon l'une quelconque des revendications 1 à 4, dans lequel la surface du logement (2) qui est dirigée vers le dessus et/ou le dessous de chaque élément tendeur (1, 1', 1") est formée de façon concave.

6. Dispositif de fixation selon la revendication 1, dans lequel la denture (3) formée au niveau de l'une des surfaces de contact des logements (2) proches l'un de l'autre est complémentaire de la denture (3) formée au niveau de l'autre surface de contact des logements (2) proches l'un de l'autre.

7. Dispositif de fixation selon la revendication 1, dans lequel les moyens de fixation sont des vis corticales.

8. Dispositif de fixation selon la revendication 5, dans lequel la tête de vis de chaque vis corticale est configurée de façon conique ou bombée de telle sorte que la surface, regardant vers le logement (2), de la tête de vis est complémentaire de la surface, respectivement proche de la tête de vis, du logement (2).

9. Dispositif de fixation selon l'une quelconque des revendications 1 à 8, comportant également au moins un élément de terminaison (8) qui est configuré en forme d'anneau et qui a une surface de contact (11) réalisée avec une denture (4), dans lequel l'élément de terminaison (8) a en outre un manchon (12) qui est formé d'un seul tenant avec lui et qui peut être placé de telle sorte à travers l'un des logements (2) d'un élément tendeur (1, 1', 1") que la denture (4) de l'élément de terminaison (8) arrive de manière complémentaire en prise avec la denture (3) formée au niveau du logement (2) et que l'élément tendeur (1, 1', 1") peut être fixé à l'os par la mise en place du moyen de fixation (7) à travers le manchon (12), logé dans le logement (2) de l'élément tendeur (1, 1', 1"), de l'élément de terminaison (8).

10. Dispositif de fixation selon l'une quelconque des revendications 1 à 8, dans lequel au moins un des éléments tendeurs (1, 1', 1") comporte à la place d'un logement (2) à une extrémité dans le sens de la longueur des éléments tendeurs (1, 1', 1") un logement annulaire (16) qui sert d'élément de terminaison, dans lequel un élément enveloppant (5) et/ou un moyen de fixation (7) peut être placé en vue de la fixation à l'os.

11. Dispositif de fixation selon l'une quelconque des revendications 1 à 8, dans lequel au moins un des éléments tendeurs (1, 1', 1") est formé avec plusieurs branches, de préférence en forme de Y, d'un seul tenant autour d'un élément de logement (13) annulaire central et dans lequel chaque extrémité de branche d'un élément tendeur (1, 1', 1") à plusieurs branches de ce type comporte un logement (2) qui suit d'un seul tenant celle-ci et au niveau d'une surface de contact regardant vers la ligne médiane imaginaire de chaque élément tendeur (1, 1', 1") dans le sens de la longueur (L), une denture (3) est formée.

12. Dispositif de fixation selon l'une quelconque des revendications 1 à 8, dans lequel au moins un des éléments tendeurs (1, 1', 1") comprend à la place d'un des logements ou des deux logements (2), au moins à une extrémité des éléments tendeurs (1, 1', 1") dans le sens de la longueur, un disque de logement (14) en forme d'anneau qui est réalisé comme un disque denté ou équivalent et qui, lors de la combinaison d'éléments tendeurs (1, 1', 1"), d'éléments enveloppants (5) et/ou de moyens de fixation (7), peut être introduit entre les logements (2), formé en forme d'anneaux, d'autres éléments tendeurs (1, 1', 1") dans le but de former une combinaison en forme de Y, en forme d'étoile ou autre d'éléments tendeurs (1, 1', 1"), d'éléments enveloppants (5) et/ou de moyens de fixation (7).
